# EUROPÄISCHE PATENTSCHRIFT

(11) **EP 0 755 919 B1**
(45) Veröffentlichungstag und Bekanntmachung des Hinweises auf die Patenterteilung: **17.11.1999**
(21) Anmeldenummer: 96111665.4
(22) Anmeldetag: 19.07.1996
(51) Int. Cl.: C07C 279/22, C07D 213/65, C07D 213/70, A61K 31/155, A61K 31/44

(54) **Substituierte Zimtsäureguanidide, Verfahren zu ihrer Herstellung, ihre Verwendung als Herz-Kreislauf-Medikament oder Diagnostikum sowie sie enthaltendes Medikament**
Substituted cinnamic acid guanidides, process for their preparation, their use as cardiovascualr medicament or diagnostic agent, as well as medicament containing them
Guanidides d'acide cinnamique substitué, procédé de leur préparation, leur utilisation comme médicament cardiovasculaire ou agent diagnostique, ainsi que médicament les contenant

(30) Priorität: 26.07.1995 DE 19527305
(43) Veröffentlichungstag der Anmeldung: 29.01.1997
(73) Patentinhaber: HOECHST AKTIENGESELLSCHAFT, 65929 Frankfurt am Main (DE)
(72) Erfinder: Schwark, Jan-Robert, Dr., 65929 Frankfurt (DE); Brendel,Joachim, Dr., 61118 Bad Vilbel (DE); Kleemann, Heinz-Werner, Dr., 65474 Bischofsheim (DE); Lang, Hans-Jochen, Dr., 65719 Hofheim (DE); Weichert, Andreas, Dr., 63329 Egelsbach (DE); Albus, Udo, Dr., 61197 Florstadt (DE); Scholz, Wolfgang, Dr., 65760 Eschborn (DE)

(56) Entgegenhaltungen:
- EP-A- 0 556 672
- DE-A- 4 325 822
- US-A- 2 734 904

## Beschreibung

Die Erfindung betrifft substituierte Zimtsäureguanidide der Formel I 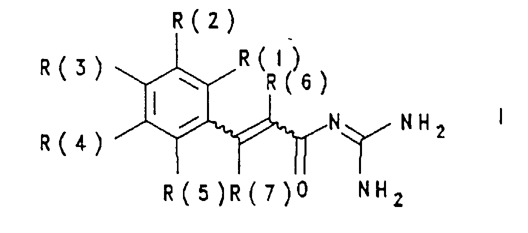 worin bedeuten:
mindestens einer der Substituenten R(1), R(2), R(3), R(4) und R(5)

-Xₐ-Y_{b}-Lₙ-U;

- X: CR(16)R(17), O, S oder NR(18);
R(16), R(17) und R(18)
unabhängig voneinander H, Alkyl mit 1, 2, 3 oder 4 C-Atomen oder Perfluoralkyl mit 1, 2, 3 oder 4 C-Atomen;
- a: Null oder 1;
- Y: Alkylen mit 1, 2, 3, 4, 5, 6, 7 oder 8 C-Atomen, Alkylen-T mit 1, 2, 3, 4, 5, 6, 7 oder 8 C-Atomen in der Alkylengruppe, T, T-Alkylen mit 1, 2, 3, 4, 5, 6, 7 oder 8 C-Atomen in der Alkylengruppe;
T NR(20), O, S oder Phenylen,
wobei das Phenylen nicht substituiert oder substituiert ist mit 1 - 3 Substituenten ausgewählt aus der Gruppe bestehend aus F, Cl, CF₃, Methyl, Methoxy und
NR(21)R(22);
R(20), R(21) und R(22)
unabhängig voneinander H, Alkyl mit 1, 2, 3 oder 4 C-Atomen oder Perfluoralkyl mit 1, 2, 3 oder 4 C-Atomen;
- b: Null oder 1;
- L: O, S, NR(23) oder CₖH₂ₖ;
- k: 1, 2, 3, 4, 5, 6, 7 oder 8;
- n: Null oder 1;
- U: NR(24)R(25) oder einen N-haltigen Heterocyclus mit 1, 2, 3, 4, 5, 6, 7, 8 oder 9 C-Atomen;
R(24) und R(25)
unabhängig voneinander Wasserstoff, Alkyl mit 1, 2, 3, 4, 5, 6, 7 oder 8 C-Atomen, Perfluoralkyl mit 1, 2, 3, 4, 5, 6, 7 oder 8 C-Atomen;
oder
R(24) und R(25)
gemeinsam 4 oder 5 Methylenguppen, von denen eine CH₂-Gruppe durch Sauerstoff, S, NH, N-CH₃ oder N-Benzyl ersetzt sein kann;
wobei die N-haltigen Heterocyclen N- oder C-verbrückt sind und nicht substituiert oder substituiert sind mit 1 - 3 Substituenten ausgewählt aus der Gruppe bestehend aus F, Cl, CF₃, Methyl, Methoxy und NR(27)R(28);
R(23), R(27) und R(28)
unabhängig voneinander H, Alkyl mit 1, 2, 3 oder 4 C-Atomen oder Perfluoralkyl mit 1, 2, 3 oder 4 C-Atomen;
und die jeweils anderen Substituenten R(1), R(2), R(3), R(4) und R(5)
unabhängig voneinander H, F, Cl, Br, I, CN, -Oₙ-CₘH₂ₘ₊₁, -Oₚ-(CH₂)ₛ-C_{q}F_{2q+1} oder -CᵣH₂ᵣR(10);
- n: Null oder 1;
- m: Null, 1, 2, 3, 4, 5, 6, 7 oder 8;
- p: Null oder 1;
- q: 1, 2, 3, 4, 5, 6, 7 oder 8;
- s: Null, 1, 2, 3 oder 4;
- r: Null, 1, 2, 3 oder 4;
- R(10): Cycloalkyl mit 3, 4, 5, 6, 7 oder 8 C-Atomen, oder Phenyl,
wobei das Phenyl nicht substituiert oder substituiert ist mit 1 - 3 Substituenten ausgewählt aus der Gruppe bestehend aus F, Cl, CF₃, Methyl, Methoxy und NR(11)R(12);
R(11) und R(12)
unabhängig voneinander H, Alkyl mit 1, 2, 3 oder 4 C-Atomen oder Perfluoralkyl mit 1, 2, 3 oder 4 C-Atomen;
R(6) und R(7)
unabhängig voneinander Wasserstoff, F, Cl, Br, l, CN, Alkyl mit 1, 2, 3, 4, 5, 6, 7 oder 8 C-Atomen, Perfluoralkyl mit 1, 2, 3, 4, 5, 6, 7 oder 8 C-Atomen, Cycloalkyl mit 3, 4, 5, 6, 7 oder 8 C-Atomen, oder Phenyl,
das nicht substituiert oder substituiert ist mit 1 - 3 Substituenten ausgewählt aus der Gruppe bestehend aus F, Cl, CF₃, Methyl, Methoxy und NR(14)R(15);
R(24) und R(15)
unabhängig voneinander H, Alkyl mit 1, 2, 3 oder 4 C-Atomen oder Perfluoralkyl mit 1, 2, 3 oder 4 C-Atomen;
sowie deren pharmazeutisch verträgliche Salze.

Bevorzugt sind Verbindungen der Formel I, in der bedeuten: mindestens einer der Substituenten R(1), R(2), R(3), R(4) und R(5)

-Xₐ-Y_{b}-Lₙ-U,

- x: CR(16)R(17), O, S oder NR(18);
R(16), R(17) und R(18)
unabhängig voneinander H, CH₃ oder CF₃;
- a: Null oder 1;
- Y: Alkylen mit 1, 2, 3, 4, 5, 6, 7 oder 8 C-Atomen, Alkylen-T mit 1, 2, 3, 4, 5, 6, 7 oder 8 C-Atomen in der Alkylengruppe, T, T-Alkylen mit 1, 2, 3, 4, 5, 6, 7 oder 8 C-Atomen in der Alkylengruppe;
T NR(20), O, S oder Phenylen,
wobei das Phenylen nicht substituiert oder substituiert ist mit 1 - 3 Substituenten ausgewählt aus der Gruppe bestehend aus F, Cl, CF₃, Methyl, Methoxy und
NR(21)R(22);
R(20), R(21) und R(22)
unabhängig voneinander H, CH₃ oder CF₃;
- b: Null oder 1;
- L: O, S, NR(23) oder CₖH₂ₖ;
- k: 1, 2, 3, 4, 5, 6, 7, 8;
- n: Null oder 1;
- u: NR(24)R(25) oder einen N-haltigen Heterocyclus mit 1, 2, 3, 4, 5, 6, 7, 8 oder 9 C-Atomen;
R(24) und R(25)
unabhängig voneinander Wasserstoff, Alkyl mit 1, 2, 3, 4, 5, 6, 7 oder 8 C-Atomen, Perfluoralkyl mit 1, 2, 3, 4, 5, 6, 7 oder 8 C-Atomen;
oder
R(24) und R(25)
gemeinsam 4 oder 5 Methylenguppen, von denen eine CH₂-Gruppe durch Sauerstoff, S, NH, N-CH₃ oder N-Benzyl ersetzt sein kann;
wobei die N-haltigen Heterocyclen N- oder C-verbrückt sind und nicht substituiert oder substituiert sind mit 1 - 3 Substituenten ausgewählt aus der Gruppe bestehend aus F, Cl, CF₃, Methyl, Methoxy und NR(27)R(28);
R(23), R(27) und R(28)
unabhängig voneinander H, CH₃ oder CF₃;
und die jeweils anderen Substituenten R(1), R(2), R(3), R(4) und R(5)
unabhängig voneinander H, F, Cl, Br, I, CN, -Oₙ-CₘH₂ₘ₊₁, -Oₚ-(CH₂)ₛ-C_{q}F_{2q+1} oder -CᵣH₂ᵣR(10),
- n: Null oder 1;
- m: Null, 1, 2, 3 oder 4;
- p: Null;
- q: 1, 2, 3, 4, 5, 6, 7 oder 8;
- s: Null;
- r: Null, 1 oder 2;
- R(10): Cycloalkyl mit 3, 4, 5, 6, 7 oder 8 C-Atomen, oder Phenyl,
wobei das Phenyl nicht substituiert oder substituiert ist mit 1 - 3 Substituenten ausgewählt aus der Gruppe bestehend aus F, Cl, CF₃, Methyl, Methoxy und NR(11)R(12);
R(11) und R(12)
unabhängig voneinander H, CH₃ oder CF₃;
R(6) und R(7)
unabhängig voneinander Wasserstoff, F, Cl, Br, I, CN, Alkyl mit 1, 2, 3 oder 4 C-Atomen, Perfluoralkyl mit 1, 2, 3 oder 4 C-Atomen, Cycloalkyl mit 3, 4, 5, 6, 7 oder 8 C-Atomen, oder Phenyl,
das nicht substituiert oder substituiert ist mit 1 - 3 Substituenten ausgewählt aus der Gruppe bestehend aus F, Cl, CF₃, Methyl, Methoxy und NR(14)R(15);
R(14) und R(15)
unabhängig voneinander H, CH₃ oder CF₃;
sowie deren pharmazeutisch verträgliche Salze.

Besonders bevorzugt sind Verbindungen der Formel I, in der bedeuten: mindestens einer der Substituenten R(2), R(3) und R(4)

-Xₐ-Y_{b}-Lₙ-U;

- X: CR(16)R(17), O, S oder NR(18);
R(16), R(17) und R(18)
unabhängig voneinander H, CH₃ oder CF₃;
- a: Null oder 1;
- Y: Alkylen mit 1, 2, 3, 4, 5, 6, 7 oder 8 C-Atomen, Alkylen-T mit 1, 2, 3, 4, 5, 6, 7 oder 8 C-Atomen in der Alkylengruppe, T, T-Alkylen mit 1, 2, 3, 4, 5, 6, 7 oder 8 C-Atomen in der Alkylengruppe;
T NR(20), 0, S oder Phenylen,
wobei das Phenylen nicht substituiert oder substituiert ist mit 1 - 3 Substituenten ausgewählt aus der Gruppe bestehend aus F, Cl, CF₃, Methyl, Methoxy und
NR(21)R(22);
R(20), R(21) und R(22)
unabhängig voneinander H, CH₃ oder CF₃;
b Null oder 1;
L O, S, NR(23) oder CₖH₂ₖ;
k 1, 2, 3, 4, 5, 6, 7, 8;
n Null oder 1;
U NR(24)R(25) oder einen N-haltigen Heterocyclus mit 1, 2, 3, 4, 5, 6, 7, 8 oder 9 C-Atomen;
R(24) und R(25)
unabhängig voneinander Wasserstoff, Alkyl mit 1, 2, 3 oder 4 C-Atomen oder CF₃;
oder
R(24) und R(25)
gemeinsam 4 oder 5 Methylenguppen, von denen eine CH₂-Gruppe durch Sauerstoff, S, NH, N-CH₃
oder N-Benzyl ersetzt sein kann;
wobei die N-haltigen Heterocyclen N- oder C-verbrückt sind und nicht substituiert oder substituiert sind mit 1 - 3 Substituenten ausgewählt aus der Gruppe bestehend aus F, Cl, CF₃, Methyl, Methoxy und NR(27)R(28);
R(23), R(27) und R(28)
unabhängig voneinander H, CH₃ oder CF₃;
und die jeweils anderen Substituenten R(1), R(2), R(3), R(4) und R(5) unabhängig voneinander H, F, Cl, Br, I, CN, -Oₙ-CₘH₂ₘ₊₁ oder CF₃;
- n: Null oder 1;
- m: Null, 1, 2, 3 oder 4;
R(6) und R(7)
unabhängig voneinander Wasserstoff, F, Cl, Br, I, CN, Alkyl mit 1, 2, 3 oder 4 C-Atomen, CF₃, Cycloalkyl mit 5, 6 oder 7 C-Atomen oder Phenyl,
das nicht substituiert oder substituiert ist mit 1 - 3 Substituenten ausgewählt aus der Gruppe bestehend aus F, Cl, CF₃, Methyl, Methoxy und NR(14)R(15);
R(14) und R(15)
unabhängig voneinander H, CH₃ oder CF₃;
sowie deren pharmazeutisch verträgliche Salze.

Ganz besonders bevorzugt sind die Verbindungen
E-3-(4-Dimethylaminophenyl)-2-methyl-propensäureguanidid,
E-3-[4-(3-Dimethylaminopropoxy)phenyl)-2-methyl-propensäureguanidid,
E-3-[4-(3-pyridyloxy)-3-(trifluormethyl)phenyl]-2-methylpropensäureguanidid,
E-3-[4-(4-pyridylthio)-3-(trifluormethyl)phenyl]-2-methylpropensäureguanidid,
E-3-[3-Cyano-4-dimethylamino-2-fluoro-phenyl)-2-methylpropensäureguanidid,
E-3-[4-(3-Dimethylaminopropoxy)phenyl)-2-fluor-propensäureguanidid,
E-3-[4-(4-(2-Dimethylaminoethylen)phenoxy)phenyl]-2-methylpropensäureguanidid,
E-3-[3,5-Difluor-4-(4-(2-dimethylaminoethylen)phenoxy)phenyl]-2-methylpropensäureguanidid,
E-3-[3,5-Difluor-4-(3-dimethylamino-phenoxy)phenyl]-2-fluorpropensäureguanidid,
E-3-[3,5-Difluor-4-(3-dimethylaminophenoxy)phenyl]-2-methylpropensäureguanidid,
E-3-[2,6-Difluor-4-(3-dimethylaminophenoxy)phenyl]-2-methylpropensäureguanidid,
E-3-[2,4-Difluor-6-(3-dimethylaminophenoxy)phenyl]-2-methylpropensäureguanidid,
sowie deren pharmazeutisch verträgliche Salze.

Enthalten die Verbindungen der Formel I ein oder mehrere Asymmetriezentren, so können diese sowohl S als auch R konfiguriert sein. Die Verbindungen können als optische Isomere, als Diastereomere, als Racemate oder als Gemische derselben vorliegen.

Die Doppelbindungsgeometrie der Verbindungen der Formel I kann sowohl E als auch Z sein. Die Verbindungen können als Doppelbindungs-lsomere im Gemisch vorliegen.

Die bezeichneten Alkylreste und Perfluoralkylreste können sowohl geradkettig als auch verzweigt vorliegen.

Als N-haltige Heterocyclen mit 1, 2, 3, 4, 5, 6, 7, 8 oder 9 C-Atomen gelten insbesondere Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Isoindolyl, Benzimidazolyl, Indazolyl, Chinolyl, Isochinolyl, Phthalazinyl, Chinoxalinyl, Chinazolinyl, Cinnolinyl.

Besonders bevorzugt sind die N-haltigen Heterocyclen Pyrrolyl, Imidazolyl, Pyrazolyl, Pyridyl, Pyrazinyl, Pyrimidinyl und Pyridazinyl.

Die Erfindung betrifft weiterhin ein Verfahren zur Herstellung der Verbindungen I, dadurch gekennzeichnet, daß man eine Verbindung der Formel II 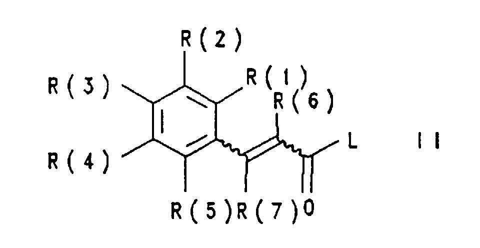 mit Guanidin umsetzt, worin R(1) bis R(7) die angegebene Bedeutung besitzen und L für eine leicht nucleophil substituierbare leaving group steht.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann.

Neben den Carbonsäurechloriden der Formel II (L = Cl) lassen sich auch weitere aktivierte Säurederivate der Formel II in an sich bekannter Weise direkt aus den zugrundeliegenden Benzoesäurederivaten (Formel II, L = OH) herstellen, wie beispielsweise die Methylester der Formel II mit L = OCH₃ durch Behandeln mit gasförmigem HCI in Methanol, die Imidazolide der Formel II durch Behandeln mit Carbonyldiimidazol [L = 1-Imidazolyl, Staab, Angew. Chem. Int. Ed. Engl. 1, 351 -367 (1962)], die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit O-[(Cyano(ethoxycarbonyl)methylen)amino]-1,1,3,3-tetramethyluroniumtetrafluoborat ("TOTU") [Proceedings of the 21. European Peptide Symposium, Peptides 1990, Editors E. Giralt and D. Andreu, Escom, Leiden, 1991]. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel II mit Guanidin erfolgt in an sich bekannter Weise in einem protischen oder aprotischen polaren aber inerten organischen Lösungsmittel. Dabei haben sich bei der Umsetzung der Benzoesäuremethylester (II, L = OMe) mit Guanidin Methanol, Isopropanol oder THF von 20°C bis zur Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von II mit Guanidin verwendet werden.

Wenn L = Cl bedeutet, arbeitet man vorteilhaft unter Zusatz eines Säurefängers, z. B. in Form von überschüssigem Guanidin zur Abbindung der Halogenwasserstoffsäure.

Ein Teil der zugrundeliegenden Benzoesäurederivate der Formel II ist bekannt und in der Literatur beschrieben. Die unbekannten Verbindungen der Formel II können nach literaturbekannten Methoden hergestellt werden. Die erhaltenen Alkenylcarbonsäuren werden nach einer der oben beschriebenen Verfahrensvarianten zu erfindungsgemäßen Verbindungen I umgesetzt.

Die Einführung einiger Substituenten gelingt durch literaturbekannte Methoden des Palladium-vermittelten cross-couplings von Arylhalogeniden bzw. Aryltriflaten mit beispielsweise Organostannanen, Organoboronsäuren oder Organoboranen oder Organokupfer- bzw. zink-verbindungen.

Carbonsäureguanidine I sind im allgemeinen schwache Basen und können Säure unter Bildung von Salzen binden. Als Säureadditionssalze kommen Salze aller pharmakologisch verträglichen Säuren infrage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind substituierte Acylguanidine.
Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid. 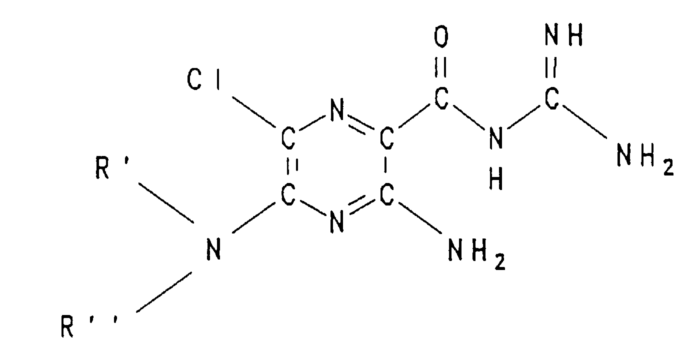
Amilorid: R',R" = H
Dimethylamilorid: R',R" = CH₃
Ethylisopropylamilorid: R'= C₂H₅, R" = CH(CH₃)₂

Darüberhinaus sind Untersuchungen bekannt geworden, die auf antiarrhythmische Eigenschaften von Amilorid hinweisen [Circulation 79, 1257 - 63 (1989)]. Einer breiten Anwendung als Antiarrhythmikum steht jedoch entgegen, daß dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten [Eur. Heart J. 9 (suppl.1): 167 (1988) (book of abstracts)].
So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

Aus WO 84/00875 sind Zimtsäureguanidide bekannt, (Rₐ und R_{c}, bzw. R_{b} und R_{d} = Doppelbindung; R(1) = substituiertes Phenyl); jedoch sind diese in allen Fällen am Guanidin zusätzlich mit Alkylgruppen substituiert, weshalb sie keine NHE-lnhibition zeigen. Außerdem wird ein basischer Substituent wie -Xₐ-Y_{b}-Z nicht beschrieben oder nahegelegt.

Aus der US-Patentschrift 2,734,904 sind Zimtsäureguanidide bekannt (R = substituiertes Phenyl, Alkyl = Alkenylen), jedoch werden ebenfalls keine basischen Substituenten wie -Xₐ-Y_{b}-Z beschrieben oder nahegelegt.

In der deutschen Patentanmeldung P 44 21 536.3 (HOE 94/F 168) Zimtsäureguanidide vorgeschlagen (x = 0, y = 0), jedoch muß einer der Substituenten R(1), R(2), R(4), R(5), R(C) oder R(D) eine Perfluoralkyl-Gruppe sein; außerdem enthalten diese Verbindungen keine basische -Xₐ-Y_{b}-Lₙ-U-Gruppe.

Aus der EP 556 672 und aus der DE 43 25 822 sind Benzoylguanidine bekannt, bei denen die Acylguanidingruppe direkt an den Phenylkern gebunden ist, die also nicht die erfindungsgemäß vorhandene olefinische Brücke aufweisen.

Die bekannten und auch die vorgeschlagenen Verbindungen entsprechen jedoch nicht allen gewünschten Anforderungen, so läßt ihre Wasserlöslichkeit noch zu wünschen übrig (Vorteil der basischen Gruppe).

Außerdem wirken Sie noch nicht im gewünschten Maße selektiv. Es war daher wünschenswert, Verbindungen mit verbesserter Wasserlöslichkeit und Selektivität zu Verfügung zu stellen.
Dies ist durch die erfindungsgemäßen Verbindungen gelungen, welche keine unerwünschten und nachteiligen salidiuretischen, jedoch sehr gute antiarrhythmische Eigenschaften aufweisen, wie sie beispielsweise für die Behandlung von Krankheiten wichtig sind, die durch Sauerstoffmangel bewirkt werden. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na+/H+-Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüberhinaus eignen sich die erfindungsgemäßen Verbindungen der Formel I ebenfalls zur Behandlung von Formen des Schocks, wie beispielweise des allergischen, cardiogenen, hypovolämischen und des bakteriellen Schocks.

Darüberhinaus zeichnen sich die erfindungsgemäßen Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.
Im Durchschnitt beträgt die tägliche Dosis einer Verbindung der Formel I bei einem etwa 75 kg schweren Patienten mindestens 0,001 mg/kg, vorzugsweise
0,01 mg/kg, bis höchstens 10 mg/kg, vorzugsweise 1 mg/kg
Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

Liste der Abkürzungen:
- MeOH: Methanol
- DMF: N,N-Dimethylformamid
- El: electron impact
- DCI: Desorption-Chemical Ionisation
- RT: Raumtemperatur
- EE: Ethylacetat (EtOAc)
- mp: Schmelzpunkt
- HEP: n-Heptan
- DME: Dimethoxyethan
- ES: Elektronenspray
- FAB: Fast Atom Bombardment
- CH₂Cl₂: Dichlormethan
- THF: Tetrahydrofuran
- eq.: Äquivalent

### Experimenteller Teil

Allgemeine Vorschriften zur Herstellung von Alkenylcarbonsäureguanididen (I)

Variante 1 A: aus Alkenylcarbonsäuren (II, L = OH)
1.0 eq. des Carbonsäurederivates der Formel II löst bzw. suspendiert man in wasserfreiem THF ( 5 ml/mmol ) und versetzt sodann mit 1.1 eq. Carbonyldiimidazol. Nach dem Rühren über 2 Stunden bei RT werden 5.0 eq. Guanidin in die Reaktionslösung eingetragen. Nach dem Rühren über Nacht destilliert man das THF unter vermindertem Druck ( im Rotationsverdampfer) ab, versetzt mit Wasser, stellt mit 2 N HCI auf pH 6 bis 7 und filtriert das entsprechende Guanidid (Formel I) ab. Die so erhaltenen Carbonsäureguanidine können durch Behandeln mit wäßriger, methanolischer oder etherischer Salzsäure oder anderen pharmakologisch verträglichen Säuren in die entsprechenden Salze übergeführt werden.

Variante 1 B: aus Alkenylcarbonsäure-alkylestern (II, L = O-Alkyl)
1.0 eq. des Carbonsäure-alkylesters der Formel II sowie 5.0 eq. Guanidin (freie Base) werden in Isopropanol gelöst oder in THF suspendiert und bis zum vollständigen Umsatz (Dünnschichtkontrolle) unter Rückfluß gekocht (typische Reaktionszeit 2 bis 5 h). Das Lösungsmittel wird unter vermindertem Druck (Rotationsverdampfer) abdestilliert in EE aufgenommen und 3 x mit NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert.
(Salzbildung vergleiche Variante A).

Beispiel 1: E-3-(4-Dimethylaminophenyl)-2-methyl-propensäureguanidid hydrochlorid 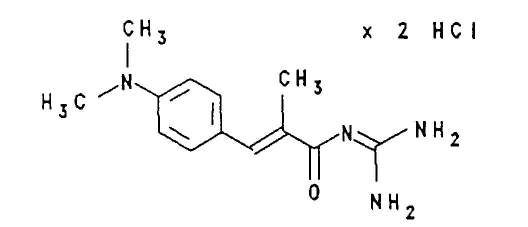
1 a) 1 eq. 2-Phosphonopropionsäuretriethylester wurde bei 0°C mit 1 eq. n-Butyllithium in Hexan deprotoniert und anschließend bei RT mit 1 eq. 4-Dimethylaminobenzaldehyd versetzt. Nach vollständiger Abreaktion des Aldehyds wurde mit Wasser aufgearbeitet und dreimal mit Toluol ausgeschüttelt. Nach Trocknen der vereinigten organischen Phasen über Magnesiumsulfat wurde das Lösungsmittel im Vakuum entfernt und das verbleibende Rohprodukt chromatographisch an Kieselgel mit EE/HEP-Gemischen als Eluens getrennt. Man isolierte E-3-(4-Dimethylaminophenyl)-2-methyl-acrylsäureethylester.
   orange-gelbliches Öl MS 233 (M⁺)
1 b) Der Ester aus 1 a) wurde gemäß einer Standardmethode (Natriumhydroxid in Methanol) verseift. Man isolierte E-3-(4-Dimethylaminophenyl)-2-methyl-acrylsäure.
   mp 190 - 194°C.
   MS : 205 (M⁺)
1 c) Die Carbonsäure aus 1 b) wurde gemäß Variante 1 A ins Zimtsäureguanidid hydrochlorid überführt.
   mp 194°C MS 247 (M+1)⁺

Beispiel 2: E-3-[4-(3-Dimethylaminopropoxy)phenyl)-2-methylpropensäureguanidid dihvdrochlorid 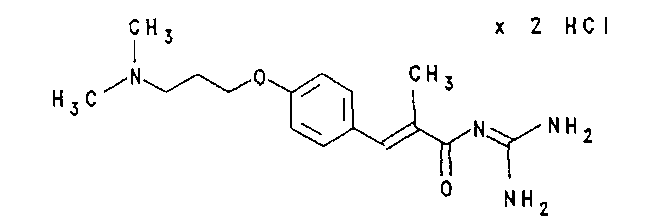
2 a) E-3-[4-(3-Dimethylaminopropoxy)phenyl)-2-methylpropensäureethylester wurde in Analogie zu Beispiel 1 a) aus 4-(3-Dimethylaminopropoxy)benzaldehyd dargestellt.
   gelbliches Öl;
   MS : 291 (M⁺)
2 b) Der Ester aus 2 a) wurde gemäß einer Standardmethode (Natriumhydroxid in Methanol) verseift. Man isolierte E-3-[4-(3-Dimethylamino-propoxy)phenyl)-2-methyl-propensäure.
   mp > 190°C.
   MS : 264 (M+1)⁺
2 c) Die Carbonsäure aus 2 b) wurde gemäß Variante 1 A ins Zimtsäureguanidid-Dihydrochlorid überführt.
   mp. 216°C MS :305 (M+1)⁺

Beispiel 3: E-3-[4-(3-pyridyloxy)-3-(trifluormethyl)phenyl]-2-methylpropensäureguanidid-dihydrochlorid 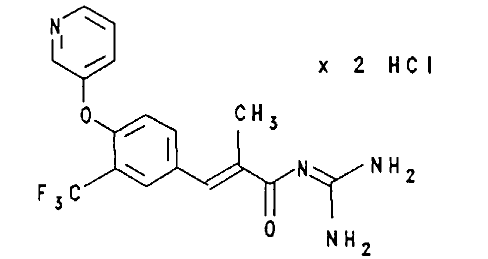
3 a) In Analogie zu Beispiel 1 a wurde E-2-Methyl-3-(4-fluor-3-trifluormethyl-phenyl)propensäureethylester aus 4-Fluor-3-trifluormethylbenzaldehyd dargestellt.
   gelbliches Öl MS:277 (M+1)⁺
3 b) Der Ester aus 3 a, 3 eq. Kaliumcarbonat und 1.1 eq. 3-Hydroxypyridin wurde in DMF 3 h unter Rückfluß erhitzt. Nach Standardaufarbeitung und Reinigung erhielt man E-3-[4-(3-pyridyloxy)-3-(trifluormethyl)phenyl]-2-methyl-propensäureethylester.
   gelbliches Öl MS:352 (M+1)⁺
3 c) Der Ester aus 3 b wurde unter Standardbedingungen in die Propensäure überführt.
   mp. 133°C MS:323 (M+1)⁺
3 d) Die Überführung der Carbonsäure aus 3 c erfolgte gemäß allgemeiner Vorschrift 1 A.
   hygroskopischer Feststoff mp. 54°C MS:365 (M+1)⁺

Beispiel 4: E-3-[4-(4-pyridylthio)-3-(trifluormethyl)phenyl]-2-methylpropensäureguanidid-dihydrochlorid 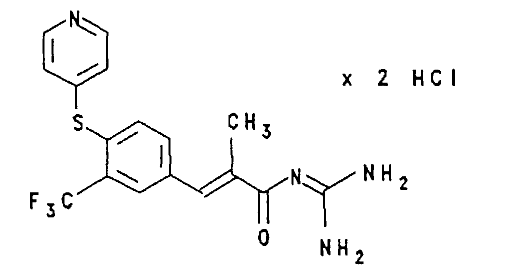
4 a) Der Ester aus 3 a wurde analog 3 b mit 4-Mercaptopyridin umgesetzt. Man isolierte E-3-[4-(4-pyridylthio)-3-(trifluormethyl)phenyl]-2-methyl-propensäureethylester.
   gelbliches Öl MS:368 (M+1)⁺
4 b) Nach Standardverseifung zur freien Carbonsäure 4 c (farbloser Feststoff, mp. > 200°C) wurde sie gemäß Variante 1 A ins Guanidid überführt.
   farblose Kristalle mp.109°C

Beispiel 5: E-3-[3-Cyano-4-dimethylamino-2-fluoro-phenyl)-2-methylpropensäureguanidid-hydrochlorid 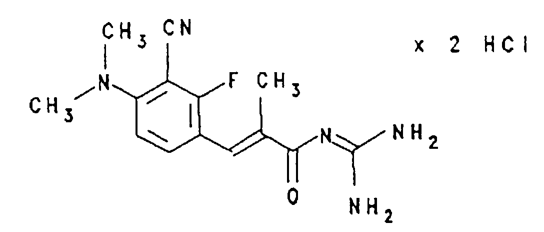 Beispiel 5 wurde in Analogie zu Beispiel 1 ausgehend vom 3-Cyano-4-dimethylamino-2-fluoro-benzaldehyd dargestellt.
E-3-[3-Cyano-4-dimethylamino-2-fluoro-phenyl)-2-methylpropensäureethylester 5 a:
   farbloses Öl MS 277 (M+1)⁺
E-3-[3-Cyano-4-dimethylamino-2-fluoro-phenyl)-2-methyl-propensäure 5 a:
   MS 249 (M+1)⁺
E-3-[3-Cyano-4-dimethylamino-2-fluoro-phenyl)-2-methylpropensäureguanidid-hydrochlorid:
   mp. 221°C, MS 290 (M+1)⁺

Beispiel 6: E-3-[4-(3-Dimethylaminopropoxy)phenyl)-2-fluor-propensäureguanidid-dihydrochlorid 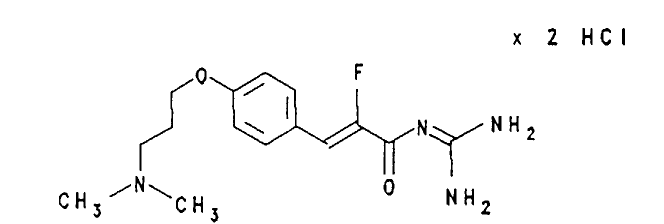
6 a) In Anlehnung an ein literaturbekanntes Verfahren (Cousseau et al., Tetrahedron Letters 34, 1993, 6903) wurde ausgehend von 4-(3-Dimethylaminopropoxy)benzaldehyd das Alpha-Fluorderivat E-3-[4-(3-Dimethylaminopropoxy)phenyl)-2-fluor-propensäureethylester synthetisiert.
   farbloses Öl MS 296 (M+1)⁺
6 b) Der Ester aus 6 a wurde gemäß Variante 1 B ins Guanidid überführt. mp. > 200°C MS 309 (M+1)⁺

Beispiel 7: E-3-[4-(4-(2-Dimethylaminoethylen)phenoxy)phenyl]-2-methylpropensäureguanidid-dihydrochlorid 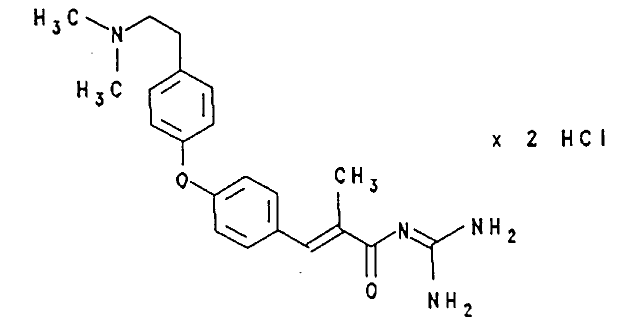
7 a) E-3-[4-Fluorphenyl]-2-methyl-propensäureethylester wurde entsprechend 1 a aus 4-Fluorbenzaldehyd dargestellt.
   farbloses Öl MS 209 (M+1)⁺
7 b) Der Ester aus 7 a, 3 eq. Kaliumcarbonat und 1.1 eq. 4-(2-Dimethylaminoethylen)phenol in DMF wurden über Nacht unter Rückfluß erhitzt. Nach Aufarbeitung und Chromatographie isolierte man E-3-[4-(4-(2-Dimethylaminoethylen)phenoxy)phenyl]-2-methylpropensäureethylester.
   farbloses Öl MS 354 (M+1)⁺
7 c) Die Verseifung des Esters aus 7 b ergab E-3-[4-(4-(2-Dimethylaminoethylen)phenoxy)phenyl]-2-methyl-propensäure. farblose Kristalle
   mp. > 220°C MS 326 (M+1)⁺
7 d) Die freie Carbonsäure aus 7 c wurde entsprechend Variante 1 A in das Guanidid überführt.
   farblose Kristalle
   mp. 170-175°C MS 367 (M+1)⁺

Beispiel 8: E-3-[3,5-Difluor-4-(4-(2-dimethylaminoethylen)phenoxy)phenyl]-2-methyl-propensäureguanididdihydrochlorid 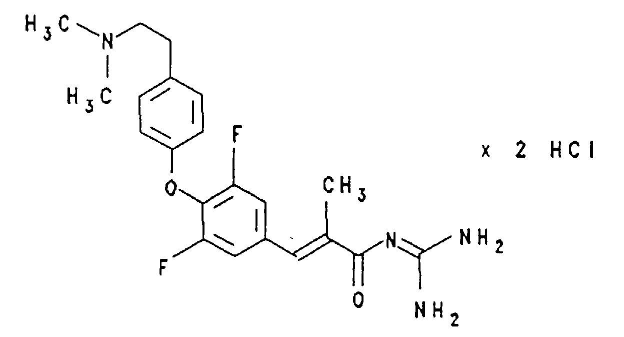
8 a) Entsprechend Beispiel 1 a wurde E-3-[3,4,5-Trifluorphenyl]-2-methylpropensäureethylester ausgehend von 3,4,5-Trifluorbenzaldehyd dargestellt.
   farbloses Öl MS 245 (M+1)⁺
8 b) Ester 8 a, 3 eq. Kaliumcarbonat und 1.1 eq. 4-(2-dimethylaminoethylen)phenol wurden in DMF 4 h bei 150-175°C gerührt. Standardaufarbeitung und Reinigung ergab E-3-[3,5-Difluor-4-(4-(2-dimethylaminoethylen)phenoxy)phenyl]-2-methyl-propensäureethylester.
   farbloses Öl MS 390 (M+1)⁺
8 c) Der Ester aus 8 b wurde gemäß Variante 1 B ins Guanidid überführt. farbloser Feststoff
   mp. > 230°C MS 403 (M+1)⁺

Beispiel 9: E-3-[3,5-Difluor-4-(4-(2-dimethylaminoethylen)phenoxy)phenyl]-2-fluor-propensäureguanididdihydrochlorid 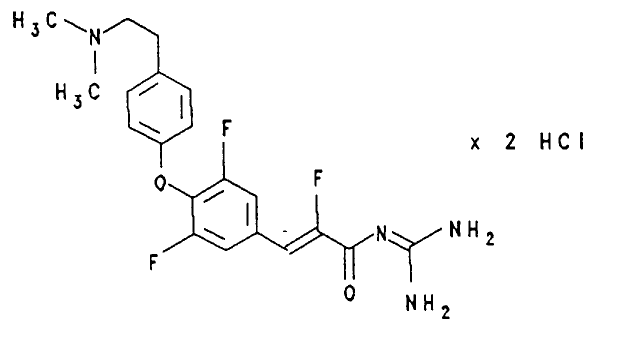
9 a) In Analogie zu Beispiel 6 wurde ausgehend von 3,4,5-Trifluorbenzaldehyd der E-3-[3,4,5-Trifluorphenyl]-2-fluorpropensäureethylester erhalten.
   farbloser Feststoff
   mp. < 55°C MS 249 (M+1)⁺
9 b) Ester 9 b, 3 eq. Kaliumcarbonat und 1.1 eq. 4-(2-Dimethylaminoethylen)phenol wurden in DMF 4 h unter Rückfluß erhitzt. Standardaufarbeitung und Reinigung ergab E-3-[3,5-Difluor-4-(4-(2-dimethylaminoethylen)phenoxy)phenyl]-2-fluor-propensäureethylester.
   farbloses Öl MS 394 (M+1)⁺
9 c) Der Ethylester aus 9 b wurde analog Variante 1 B ins Guanidid überführt.
   farbloser Feststoff
   mp. 215°C MS 407 (M+1)⁺

Beispiel 10: E-3-[3,5-Difluor-4-(3-dimethylaminophenoxy)phenyl]-2-methyl-propensäureguanidid-hydrochlorid 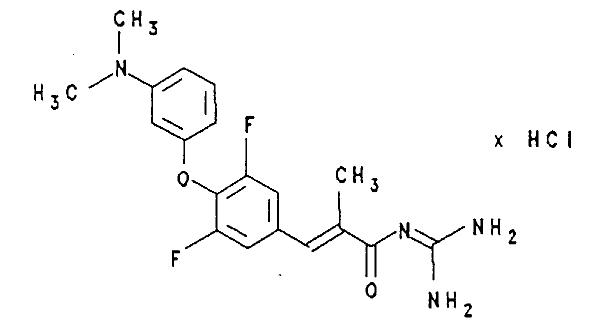
10 a) Der Ester aus 8 a, 3 eq. Kaliumcarbonat und 1.1 eq. 3-Dimethylaminophenol wurden in DMF 5 h bei 150°C gerührt. Nach Aufarbeitung und Reinigung isolierte man E-3-[3,5-Difluor-4-(3-dimethylaminophenoxy)phenyl]-2-methyl-propensäureethylester. farbloses Öl MS 362 (M+1(⁺
10 b) Der Ester aus 10 a wurde gemäß Variante 1 B ins Guanidid überführt.
   mp. 150-160°C MS 375 (M+1(⁺

Beispiel 11 und 12: E-3-[2,6-Difluor-4-(3-dimethylaminophenoxy)phenyl]-2-methyl-propensäureguanidid und E-3-[2,4-Difluor-6-(3-dimethylaminophenoxy)-phenyl]-2-methyl-propensäureguanidid 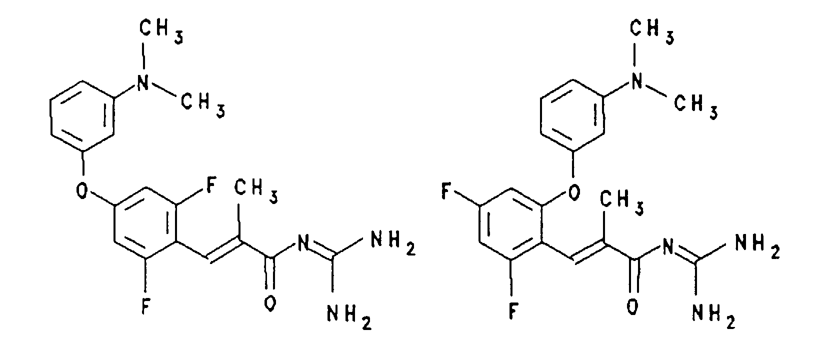
11 a/12 a) In Analogie zu 8 a wurde ausgehend von 2,4,6-Trifluorbenzaldehyd der E-3-[2,4,6-Trifluorphenyl]-2-methylpropensäureethylester synthetisiert.
   farbloses Öl MS MS 245 (M+1(⁺
11 b/12 b) Der Ester aus 11 a/12 a, 3 eq. Kaliumcarbonat und 1.1 eq. 3-Dimethylaminophenol wurden in DMF 3 h bei 150°C gerührt. Man isolierte ein Isomerengemisch der Ethylester E-3-[2,6-Difluor-4-(3-dimethylaminophenoxy)phenyl]-2-methyl-propensäureester und E-3-[2,4-Difluor-6-(3-dimethylaminophenoxy)-phenyl]-2-methyl-propensäureester. farbloses Öl
   MS 362 (M+1)⁺
11 c/12 c) Der Gemisch der Ester aus 11 b/12 b wurde gemäß Variante 1 B in die jeweiligen Guanidide überführt und als Gemisch isoliert.
   Feststoff MS 375 (M+1)⁺

### Pharmakologische Daten:

### Inhibitoren des Na Na⁺/H⁺-Exchangers von Kaninchenerythozyten:

Weiße Neuseeland-Kaninchen (Ivanovas) erhielten eine Standard-Diät mit 2% Cholesterin für sechs Wochen, um den Na Na⁺/H⁺-Austausch zu aktivieren und so den Na⁺-Influx in die Erythrozyten via Na⁺/H⁺-Austausch flammenphotometrisch bestimmen zu können. Das Blut wurde den Ohrarterien entnommen und durch 25 IE/ml Kalium-Heparin ungerinnbar gemacht. Ein Teil jeder Probe wurde zur Doppelbestimmung des Hämatokrits durch Zentrifugation benutzt. Aliquots von jeweils 100 µl dienten zur Messung des Na⁺-Ausgangsgehalts der Erythrozyten.

Um den Amilorid-sensitiven Natrium-lnflux zu bestimmen, wurden 100 µl jeder Blutprobe in jeweils 5 ml eines hyperosmolaren Salz-Sucrose-Mediums (mmol/l: 140 NaCl, 3 KCI, 150 Sucrose, 0,1 Ouabain, 20 Tris-hydroxymethyl-aminomethan) bei pH 7,4 und 37°C inkubiert. Die Erythrozyten wurden danach dreimal mit eiskalter MgCl₂-Ouabain-Lösung (mmol/l: 112 MgCl₂, 0,1 Ouabain) gewaschen und in 2,0 ml destilliertem Wasser hämolysiert. Der intrazelluläre Natriumgehalt wurde flammenphotometrisch bestimmt.

Der Na⁺-Nettoinflux wurde aus der Differenz zwischen Natrium-Ausgangswerten und dem Natriumgehalt der Erythrozyten nach Inkubation errechnet. Der Amilorid-hemmbare Natrium-lnflux ergab sich aus der Differenz des Natriumgehalts der Erythrozyten nach Inkubation mit und ohne Amilorid 3 x 10⁻⁴ mol/l. Auf diese Weise wurde auch bei den erfindungsgemäßen Verbindungen verfahren.

Ergebnisse zur Inhibition des Na⁺/H⁺-Exchangers:

| Beispiel | IC₅₀ [µmol/l] |
|---|---|
| 1 | < 1 |
| 2 | < 1 |

## Patentansprüche

1. Substituierte Zimtsäureguanidide der Formel I 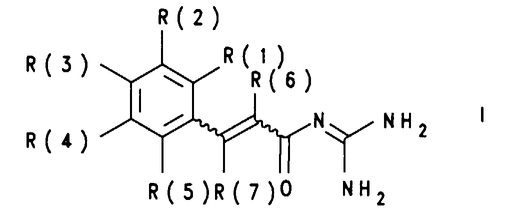 worin bedeuten:
mindestens einer der Substituenten R(1), R(2), R(3), R(4) und R(5)
-Xₐ-Y_{b}-Lₙ-U;
X CR(16)R(17), O, S oder NR(18);
R(16), R(17) und R(18)
unabhängig voneinander H, Alkyl mit 1, 2, 3 oder 4 C-Atomen oder Perfluoralkyl mit 1, 2, 3 oder 4 C-Atomen;
a Null oder 1;
Y Alkylen mit 1, 2, 3, 4, 5, 6, 7 oder 8 C-Atomen, Alkylen-T mit 1, 2, 3, 4, 5, 6, 7 oder 8 C-Atomen in der Alkylengruppe, T, T-Alkylen mit 1, 2, 3, 4, 5, 6, 7 oder 8 C-Atomen in der Alkylengruppe;
T NR(20), O, S oder Phenylen,
wobei das Phenylen nicht substituiert oder substituiert ist mit 1 - 3 Substituenten ausgewählt aus der Gruppe bestehend aus F, Cl, CF₃, Methyl, Methoxy und NR(21)R(22);
R(20), R(21) und R(22)
unabhängig voneinander H, Alkyl mit 1, 2, 3 oder 4 C-Atomen oder Perfluoralkyl mit 1, 2, 3 oder 4 C-Atomen;
b Null oder 1;
L O, S, NR(23) oder cₖH₂ₖ;
k 1, 2, 3, 4, 5, 6, 7 oder 8;
n Null oder 1;
U NR(24)R(25) oder einen N-haltigen Heterocyclus mit 1, 2, 3, 4, 5, 6, 7, 8 oder 9 C-Atomen;
R(24) und R(25)
unabhängig voneinander Wasserstoff, Alkyl mit 1, 2, 3, 4, 5, 6, 7 oder 8 C-Atomen, Perfluoralkyl mit 1, 2, 3, 4, 5, 6, 7 oder 8 C-Atomen;
oder
R(24) und R(25)
gemeinsam 4 oder 5 Methylenguppen, von denen eine CH₂-Gruppe durch Sauerstoff, S, NH, N-CH₃ oder N-Benzyl ersetzt sein kann;
wobei die N-haltigen Heterocyclen N- oder C-verbrückt sind und nicht substituiert oder substituiert sind mit 1 - 3 Substituenten ausgewählt aus der Gruppe bestehend aus F, Cl, CF₃, Methyl, Methoxy und NR(27)R(28);
R(23), R(27) und R(28)
unabhängig voneinander H, Alkyl mit 1, 2, 3 oder 4 C-Atomen oder Perfluoralkyl mit 1, 2, 3 oder 4 C-Atomen;
und die jeweils anderen Substituenten R(1), R(2), R(3), R(4) und R(5)
unabhängig voneinander H, F, Cl, Br, I, CN, -Oₙ-CₘH_{2m + 1}, -Oₚ-(CH₂)ₛ-C_{q}F_{2q + 1} oder -CᵣH₂ᵣR(10);
n Null oder 1;
m Null, 1, 2, 3, 4, 5, 6, 7 oder 8;
p Null oder 1;
q 1, 2, 3, 4, 5, 6, 7 oder 8;
s Null, 1, 2, 3 oder 4;
r Null, 1, 2, 3 oder 4;
R(10) Cycloalkyl mit 3, 4, 5, 6, 7 oder 8 C-Atomen, oder Phenyl,
wobei das Phenyl nicht substituiert oder substituiert ist mit 1 - 3 Substituenten ausgewählt aus der Gruppe bestehend aus F, Cl, CF₃, Methyl, Methoxy und NR(11)R(12);
R(11) und R(12)
unabhängig voneinander H, Alkyl mit 1, 2, 3 oder 4 C-Atomen oder Perfluoralkyl mit 1, 2, 3 oder 4 C-Atomen;
R(6) und R(7)
unabhängig voneinander Wasserstoff, F, Cl, Br, I, CN, Alkyl mit 1, 2, 3, 4, 5, 6, 7 oder 8 C-Atomen, Perfluoralkyl mit 1, 2, 3, 4, 5, 6, 7 oder 8 C-Atomen, Cycloalkyl mit 3, 4, 5, 6, 7 oder 8 C-Atomen, oder Phenyl,
das nicht substituiert oder substituiert ist mit 1 - 3 Substituenten ausgewählt aus der Gruppe bestehend aus F, Cl, CF₃, Methyl, Methoxy und NR(14)R(15);
R(14) und R(15)
unabhängig voneinander H, Alkyl mit 1, 2, 3 oder 4 C-Atomen oder Perfluoralkyl mit 1, 2, 3 oder 4 C-Atomen;
sowie deren pharmazeutisch verträgliche Salze.

2. Verbindung der Formel I nach Anspruch 1, dadurch gekennzeichnet, daß darin bedeuten:
mindestens einer der Substituenten R(1), R(2), R(3), R(4) und R(5)
-Xₐ-Y_{b}-Lₙ-U;
X CR(16)R(17), O, S oder NR(18);
R(16), R(17) und R(18)
unabhängig voneinander H, CH₃ oder CF₃;
a Null oder 1;
Y Alkylen mit 1, 2, 3, 4, 5, 6, 7 oder 8 C-Atomen, Alkylen-T mit 1, 2, 3, 4, 5, 6, 7 oder 8 C-Atomen in der Alkylengruppe, T, T-Alkylen mit 1, 2, 3, 4, 5, 6, 7 oder 8 C-Atomen in der Alkylengruppe;
T NR(20), O, S oder Phenylen,
wobei das Phenylen nicht substituiert oder substituiert ist mit 1 - 3 Substituenten ausgewählt aus der Gruppe bestehend aus F, Cl, CF₃, Methyl, Methoxy und NR(21)R(22);
R(20), R(21) und R(22)
unabhängig voneinander H, CH₃ oder CF₃;
b Null oder 1;
L O, S, NR(23) oder CₖH₂ₖ;
k 1, 2, 3, 4, 5, 6, 7, 8;
n Null oder 1;
U NR(24)R(25) oder einen N-haltigen Heterocyclus mit 1, 2, 3, 4, 5, 6, 7, 8 oder 9 C-Atomen;
R(24) und R(25)
unabhängig voneinander Wasserstoff, Alkyl mit 1, 2, 3, 4, 5, 6, 7 oder 8 C-Atomen, Perfluoralkyl mit 1, 2, 3, 4, 5, 6, 7 oder 8 C-Atomen;
oder
R(24) und R(25)
gemeinsam 4 oder 5 Methylenguppen, von denen eine CH₂-Gruppe durch Sauerstoff, S, NH, N-CH₃ oder N-Benzyl ersetzt sein kann;
wobei die N-haltigen Heterocyclen N- oder C-verbrückt sind und nicht substituiert oder substituiert sind mit 1 - 3 Substituenten ausgewählt aus der Gruppe bestehend aus F, Cl, CF₃, Methyl, Methoxy und NR(27)R(28);
R(23), R(27) und R(28)
unabhängig voneinander H, CH₃ oder CF₃;
und die jeweils anderen Substituenten R(1), R(2), R(3), R(4) und R(5) unabhängig voneinander H, F, Cl, Br, I, CN, -Oₙ-CₘH_{2m + 1}, -Oₚ-(CH₂)ₛ-C_{q}F_{2q + 1} oder -CᵣH₂ᵣR(10);
n Null oder 1;
m Null, 1, 2, 3 oder 4;
p Null;
q 1, 2, 3, 4, 5, 6, 7 oder 8;
s Null;
r Null, 1 oder 2;
R(10) Cycloalkyl mit 3, 4, 5, 6, 7 oder 8 C-Atomen, oder Phenyl,
wobei das Phenyl nicht substituiert oder substituiert ist mit 1 - 3 Substituenten ausgewählt aus der Gruppe bestehend aus F, Cl, CF₃, Methyl, Methoxy und NR(11)R(12);
R(11) und R(12)
unabhängig voneinander H, CH₃ oder CF₃;
R(6) und R(7)
unabhängig voneinander Wasserstoff, F, Cl, Br, I, CN, Alkyl mit 1, 2, 3 oder 4 C-Atomen, Perfluoralkyl mit 1, 2, 3 oder 4 C-Atomen, Cycloalkyl mit 3, 4, 5, 6, 7 oder 8 C-Atomen, oder Phenyl,
das nicht substituiert oder substituiert ist mit 1 - 3 Substituenten ausgewählt aus der Gruppe bestehend aus F, Cl, CF₃, Methyl, Methoxy und NR(14)R(15);
R(14) und R(15)
unabhängig voneinander H, CH₃ oder CF₃.

3. Verbindungen der Formel I nach einem der Ansprüche 1 oder 2, dadurch gekennzeichnet, daß darin bedeuten:
mindestens einer der Substituenten R(2), R(3) und R(4)
-Xₐ-Y_{b}-Lₙ-U;
X CR(16)R(17), O, S oder NR(18);
R(16), R(17) und R(18)
unabhängig voneinander H, CH₃ oder CF₃;
a Null oder 1;
Y Alkylen mit 1, 2, 3, 4, 5, 6, 7 oder 8 C-Atomen, Alkylen-T mit 1, 2, 3, 4, 5, 6, 7 oder 8 C-Atomen in der Alkylengruppe, T, T-Alkylen mit 1, 2, 3, 4, 5, 6, 7 oder 8 C-Atomen in der Alkylengruppe;
T NR(20), O, S oder Phenylen,
wobei das Phenylen nicht substituiert oder substituiert ist mit 1 - 3 Substituenten ausgewählt aus der Gruppe bestehend aus F, Cl, CF₃, Methyl, Methoxy und NR(21)R(22);
R(20), R(21) und R(22)
unabhängig voneinander H, CH₃ oder CF₃;
b Null oder 1;
L O, S, NR(23) oder CₖH₂ₖ;
k 1, 2, 3, 4, 5, 6, 7, 8;
n Null oder 1;
U NR(24)R(25) oder einen N-haltigen Heterocyclus mit 1, 2, 3, 4, 5, 6, 7, 8 oder 9 C-Atomen;
R(24) und R(25)
unabhängig voneinander Wasserstoff, Alkyl mit 1, 2, 3 oder 4 C-Atomen oder CF₃;
oder
R(24) und R(25)
gemeinsam 4 oder 5 Methylenguppen, von denen eine CH₂-Gruppe durch Sauerstoff, S, NH, N-CH3 oder N-Benzyl ersetzt sein kann;
wobei die N-haltigen Heterocyclen N- oder C-verbrückt sind und nicht substituiert oder substituiert sind mit 1 - 3 Substituenten ausgewählt aus der Gruppe bestehend aus F, Cl, CF₃, Methyl, Methoxy und NR(27)R(28);
R(23), R(27) und R(28)
unabhängig voneinander H, CH₃ oder CF₃;
und die jeweils anderen Substituenten R(1), R(2), R(3), R(4) und R(5) unabhängig voneinander H, F, Cl, Br, I, CN, -Oₙ-CₘH_{2m + 1} oder CF₃;
n Null oder 1;
m Null 1, 2, 3 oder 4;
R(6) und R(7)
unabhängig voneinander Wasserstoff, F, Cl, Br, I, CN, Alkyl mit 1, 2, 3 oder 4 C-Atomen, CF₃, Cycloalkyl mit 5, 6 oder 7 C-Atomen oder Phenyl,
das nicht substituiert oder substituiert ist mit 1 - 3 Substituenten ausgewählt aus der Gruppe bestehend aus F, Cl, CF₃, Methyl, Methoxy und NR(14)R(15);
R(14) und R(15)
unabhängig voneinander H, CH₃ oder CF₃.

4. Verbindung der Formel I nach einem der Ansprüche 1 bis 3, dadurch gekennzeichnet, daß sie ausgewählt sind aus der Gruppe bestehend aus
E-3-(4-Dimethylaminophenyl)-2-methyl-propensäureguanidid,
E-3-[4-(3-Dimethylaminopropoxy)phenyl)-2-methyl-propensäureguanidid,
E-3-[4-(3-pyridyloxy)-3-(trifluormethyl)phenyl]-2-methyl-propensäureguanidid,
E-3-[4-(4-pyridylthio)-3-(trifluormethyl)phenyl]-2-methyl-propensäureguanidid,
E-3-[3-Cyano-4-dimethylamino-2-fluoro-phenyl)-2-methyl-propensäureguanidid,
E-3-[4-(3-Dimethylaminopropoxy)phenyl)-2-fluor-propensäureguanidid,
E-3-[4-(4-(2-Dimethylaminoethylen)phenoxy)phenyl]-2-methylpropensäureguanidid,
E-3-[3,5-Difluor-4-(4-(2-dimethylaminoethylen)phenoxy)phenyl]-2-methylpropensäureguanidid,
E-3-[3,5-Difluor-4-(3-dimethylamino-phenoxy)phenyl]-2-fluorpropensäureguanidid,
E-3-[3,5-Difluor-4-(3-dimethylaminophenoxy)phenyl]-2-methylpropensäureguanidid,
E-3-[2,6-Difluor-4-(3-dimethylaminophenoxy)phenyl]-2-methylpropensäureguanidid,
E-3-[2,4-Difluor-6-(3-dimethylaminophenoxy)phenyl]-2-methylpropensäureguanidid.

5. Verfahren zur Herstellung einer Verbindung I nach Anspruch 1, dadurch gekennzeichnet daß man eine Verbindung der Formel II 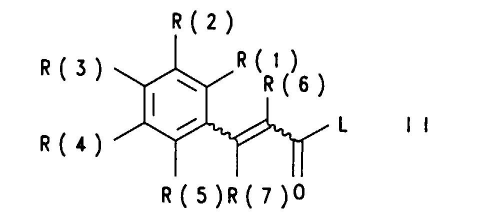 worin R(1) bis R(7) wie in Anspruch 1 definiert sind und L für Methoxy, Phenoxy, Phenylthio, Methylthio, 2-Pyridylthio, Imdiazolyl, -OCOOEthyl, -O-SO₂-Tolyl, -O-C(= N-Cyclohexyl)-(NH-Cyclohexyl) oder -O-C⁺[N(CH₃)₂][N(CH₃)₂] steht, mit Guanidin umsetzt.

6. Verwendung einer Verbindung I nach Anspruch 1 zur Herstellung eines Medikaments zu Behandlung von Arrhythmien.

7. Verwendung einer Verbindung I nach Anspruch 1 zur Herstellung eines Medikaments zur Behandlung oder Prophylaxe des Herzinfarkts.

8. Verwendung einer Verbindung I nach Anspruch 1 zur Herstellung eines Medikaments zur Behandlung oder Prophylaxe der Angina Pectoris.

9. Verwendung einer Verbindung I nach Anspruch 1 zur Herstellung eines Medikaments zur Behandlung oder Prophylaxe von ischämischen Zuständen des Herzens.

10. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

11. Verwendung einer Verbindung I nach Anspruch 1 zur Herstellung eines Medikaments zur Behandlung oder Prophylaxe von ischämischen Zuständen peripherer Organe und Gliedmaßen.

12. Verwendung einer Verbindung I nach Anspruch 1 zur Herstellung eines Medikaments zur Behandlung von Schockzuständen.

13. Verwendung einer Verbindung I nach Anspruch 1 zur Herstellung eines Medikaments zum Einsatz bei chirurgischen Operationen und Organtransplantationen.

14. Verwendung einer Verbindung I nach Anspruch 1 zur Herstellung eines Medikaments zur Konservierung und Lagerung von Transplantaten für chirurgische Maßnahmen.

15. Verwendung einer Verbindung I nach Anspruch I zur Herstellung eines Medikaments zur Behandlung von Krankheiten, bei denen die Zellproliferation eine primäre oder sekundäre Ursache darstellt.

16. Arzneimittel, gekennzeichnet durch einen wirksamen Gehalt einer Verbindung der Formel I nach einem oder mehreren der Ansprüche 1 bis 3.

## Claims

1. A substituted cinnamic acid guanidide of the formula I 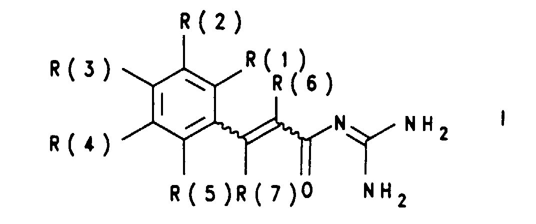 in which: at least one of the substituents R(1), R(2), R(3), R(4) and R(5) is
-Xₐ-Y_{b}-Lₙ-U;
X is CR(16)R(17), O, S or NR(18);
R(16), R(17) and R(18)
independently of one another are H, alkyl having 1, 2, 3 or 4 carbon atoms or perfluoroalkyl having 1, 2, 3 or 4 carbon atoms;
a is zero or 1;
Y is alkylene having 1, 2, 3, 4, 5, 6, 7 or 8 carbon atoms, alkylene-T having 1, 2, 3, 4, 5, 6, 7 or 8 carbon atoms in the alkylene group, T or T-alkylene having 1, 2, 3, 4, 5, 6, 7 or 8 carbon atoms in the alkylene group;
T is NR(20), O, S or phenylene, where the phenylene is unsubstituted or substituted by 1 - 3 substituents chosen from the group consisting of F, Cl, CF₃, methyl, methoxy and NR(21)R(22);
R(20), R(21) and R(22)
independently of one another are H, alkyl having 1, 2, 3 or 4 carbon atoms or perfluoroalkyl having 1, 2, 3 or 4 carbon atoms;
b is zero or 1;
L is O, S, NR(23) or CₖH₂ₖ;
k is 1, 2, 3, 4, 5, 6, 7 or 8;
n is zero or 1;
U is NR(24)R(25) or an N-containing heterocyclic radical having 1, 2, 3, 4, 5, 6, 7, 8 or 9 carbon atoms;
R(24) and R(25)
independently of one another are hydrogen, alkyl having 1, 2, 3, 4, 5, 6, 7 or 8 carbon atoms or perfluoroalkyl having 1, 2, 3, 4, 5, 6, 7 or 8 carbon atoms;
or
R(24) and R(25)
together are 4 or 5 methylene groups, one CH₂ group of which can be replaced by oxygen, S, NH, N-CH₃ or N-benzyl;
where the N-containing heterocyclic radicals are N- or C-bridged and are unsubstituted or substituted by 1 - 3 substituents chosen from the group consisting of F, Cl, CF₃, methyl, methoxy and NR(27)R(28);
R(23), R(27) and R(28)
independently of one another are H, alkyl having 1, 2, 3 or 4 carbon atoms or perfluoroalkyl having 1, 2, 3 or 4 carbon atoms;
and each of the other substituents R(1), R(2), R(3), R(4) and R(5) independently of one another are H, F, Cl, Br, I, CN, -Oₙ-CₘH₂ₘ₊₁,-Oₚ-(CH₂)ₛ-C_{q}F_{2q+1} or -CᵣH₂ᵣR(10);
n is zero or 1;
m is zero, 1, 2, 3, 4, 5, 6, 7 or 8;
p is zero or 1;
q is 1, 2, 3, 4, 5, 6, 7 or 8;
s is zero, 1, 2, 3 or 4;
r is zero, 1, 2, 3 or 4;
R(10) is cycloalkyl having 3, 4, 5, 6, 7 or 8 carbon atoms, or phenyl, where the phenyl is unsubstituted or substituted by 1 - 3 substituents chosen from the group consisting of F, Cl, CF₃, methyl, methoxy and NR(11)R(12);
R(11) und R(12)
independently of one another are H, alkyl having 1, 2, 3 or 4 carbon atoms or perfluoroalkyl having 1, 2, 3 or 4 carbon atoms;
R(6) and R(7)
independently of one another are hydrogen, F, Cl, Br, I, CN, alkyl having 1, 2, 3, 4, 5, 6, 7 or 8 carbon atoms, perfluoroalkyl having 1, 2, 3, 4, 5, 6, 7 or 8 carbon atoms, cycloalkyl having 3, 4, 5, 6, 7 or 8 carbon atoms, or phenyl,
which is unsubstituted or substituted by 1 - 3 substituents chosen from the group consisting of F, Cl, CF₃, methyl, methoxy and NR(14)R(15);
R(14) and R(15)
independently of one another are H, alkyl having 1, 2, 3 or 4 carbon atoms or perfluoroalkyl having 1, 2, 3 or 4 carbon atoms; or a pharmaceutically tolerated salt thereof.

2. A compound of the formula I as claimed in claim 1, wherein:
at least one of the substituents R(1), R(2), R(3), R(4) and R(5) is
-Xₐ-Y_{b}-Lₙ-U;
X is CR(16)R(17), O, S or NR(18);
R(16), R(17) and R(18)
independently of one another are H, CH₃ or CF₃;
a is zero or 1;
Y is alkylene having 1, 2, 3, 4, 5, 6, 7 or 8 carbon atoms, alkylene-T having 1, 2, 3, 4,-5, 6, 7 or 8 carbon atoms in the alkylene group, T or T-alkylene having 1, 2, 3, 4, 5, 6, 7 or 8 carbon atoms in the alkylene group;
T is NR(20), O, S or phenylene,
where the phenylene is unsubstituted or substituted by 1 - 3 substituents chosen from the group consisting of F, Cl, CF₃, methyl, methoxy and NR(21)R(22);
R(20), R(21) and R(22)
independently of one another are H, CH₃ or CF₃;
b is zero or 1;
L is O, S, NR(23) or CₖH₂ₖ;
k is 1, 2, 3, 4, 5, 6, 7, 8;
n is zero or 1;
U is NR(24)R(25) or an N-containing heterocyclic radical having 1, 2, 3, 4, 5, 6, 7, 8 or 9 carbon atoms;
R(24) and R(25)
independently of one another are hydrogen, alkyl having 1, 2, 3, 4, 5, 6, 7 or 8 carbon atoms, perfluoroalkyl having 1, 2, 3, 4, 5, 6, 7 or 8 carbon atoms;
or
R(24) and R(25)
together are 4 or 5 methylene groups, one CH₂ group of which can be replaced by oxygen, S, NH, N-CH₃ or N-benzyl;
where the N-containing heterocyclic radicals are N- or C-bridged and are unsubstituted or substituted by 1 - 3 substituents chosen from the group consisting of F, Cl, CF₃, methyl, methoxy and NR(27)R(28);
R(23), R(27) and R(28)
independently of one another are H, CH₃ or CF₃;
and each of the other substituents R(1), R(2), R(3), R(4) and R(5)
independently of one another are H, F, Cl, Br, I, CN, -Oₙ-CₘH₂ₘ₊₁, -Oₚ-(CH₂)ₛ-C_{q}F_{2q+1} or -CᵣH₂ᵣR(10);
n is zero or 1;
m is zero, 1, 2, 3 or 4;
p is zero;
q is 1, 2, 3, 4, 5, 6, 7 or 8;
s is zero;
r is zero, 1 or 2;
R(10) is cycloalkyl having 3, 4, 5, 6, 7 or 8 carbon atoms, or phenyl,
where the phenyl is unsubstituted or substituted by 1 - 3 substituents chosen from the group consisting of F, Cl, CF₃, methyl, methoxy and NR(11)R(12); R(11) and R(12)
independently of one another are H, CH₃ or CF₃;
R(6) and R(7)
independently of one another are hydrogen, F, Cl, Br, I, CN, alkyl having 1, 2, 3 or 4 carbon atoms, perfluoroalkyl having 1, 2, 3 or 4 carbon atoms, cycloalkyl having 3, 4, 5, 6, 7 or 8 carbon atoms, or phenyl,
which is unsubstituted or substituted by 1 - 3 substituents chosen from the group consisting of F, Cl, CF₃, methyl, methoxy and NR(14)R(15);
R(14) and R(15)
independently of one another are H, CH₃ or CF₃.

3. A compound of the formula I as claimed in one of claims 1 or 2, wherein: at least one of the substituents R(2), R(3) and R(4) is
-Xₐ-Y_{b}-Lₙ-U;
X is CR(16)R(17), O, S or NR(18);
R(16), R(17) and R(18)
independently of one another are H, CH₃ or CF₃;
a is zero or 1;
Y is alkylene having 1, 2, 3, 4, 5, 6, 7 or 8 carbon atoms, alkylene-T having 1, 2, 3, 4, 5, 6, 7 or 8 carbon atoms in the alkylene group, T or T-alkylene having 1, 2, 3, 4, 5, 6, 7 or 8 carbon atoms in the alkylene group;
T is NR(20), O, S or phenylene,
where the phenylene is unsubstituted or substituted by 1 - 3 substituents chosen from the group consisting of F, Cl, CF₃, methyl, methoxy and NR(21)R(22);
R(20), R(21) and R(22)
independently of one another are H, CH₃ or CF₃;
b is zero or 1;
L is O, S, NR(23) or CₖH₂ₖ;
k is 1, 2, 3, 4, 5, 6, 7, 8;
n is zero or 1;
U is NR(24)R(25) or an N-containing heterocyclic radical having 1, 2, 3, 4, 5, 6, 7, 8 or 9 carbon atoms;
R(24) and R(25)
independently of one another are hydrogen, alkyl having 1, 2, 3 or 4 carbon atoms or CF₃;
or
R(24) and R(25)
together are 4 or 5 methylene groups, one CH₂ group of which can be replaced by oxygen, S, NH, N-CH₃ or N-benzyl;
where the N-containing heterocyclic radicals are N- or C-bridged and are unsubstituted or substituted by 1 - 3 substituents chosen from the group consisting of F, Cl, CF₃, methyl, methoxy and NR(27)R(28);
R(23), R(27) and R(28)
independently of one another are H, CH₃ or CF₃;
and each of the other substituents R(1), R(2), R(3), R(4) and R(5)
independently of one another are H, F, Cl, Br, I, CN, -Oₙ-CₘH₂ₘ₊₁ or CF₃;
n is zero or 1;
m is zero, 1, 2, 3 or 4;
R(6) and R(7)
independently of one another are hydrogen, F, Cl, Br, I, CN, alkyl having 1, 2, 3 or 4 carbon atoms, CF₃, cycloalkyl having 5, 6 or 7 carbon atoms or phenyl,
which is unsubstituted or substituted by 1 - 3 substituents chosen from the group consisting of F, Cl, CF₃, methyl, methoxy and NR(14)R(15);
R(14) and R(15)
independently of one another are H, CH₃ or CF₃.

4. A compound of the formula I as claimed in one of claims 1 to 3, which is chosen from the group consisting of
E-3-(4-dimethylaminophenyl)-2-methyl-propenoic acid guanidide,
E-3-[4-(3-dimethylaminopropoxy)phenyl)-2-methyl-propenoic acid guanidide,
E-3-[4-(3-pyridyloxy)-3-(trifluoromethyl)phenyl]-2-methyl-propenoic acid guanidide,
E-3-[4-(4-pyridylthio)-3-(trifluoromethyl)phenyl]-2-methyl-propenoic acid guanidide,
E-3-(3-cyano-4-dimethylamino-2-fluoro-phenyl)-2-methyl-propenoic acid guanidide,
E-3-[4-(3-dimethylaminopropoxy)phenyl]-2-fluoro-propenoic acid guanidide,
E-3-[4-(4-(2-dimethylaminoethylene)phenoxy)phenyl]-2-methyl-propenoic acid guanidide,
E-3-[3,5-difluoro-4-(4-(2-dimethylaminoethylene)phenoxy)phenyl]-2-methylpropenoic acid guanidide,
E-3-[3,5-difluoro-4-(3-dimethylamino-phenoxy)phenyl]-2-fluoro-propenoic acid guanidide,
E-3-[3,5-difluoro-4-(3-dimethylaminophenoxy)phenyl]-2-methyl-propenoic acid guanidide,
E-3-[2,6-difluoro-4-(3-dimethylaminophenoxy)phenyl]-2-methyl-propenoic acid guanidide, and
E-3-[2,4-difluoro-6-(3-dimethylaminophenoxy)phenyl]-2-methyl-propenoic acid guanidide.

5. A process for the preparation of a compound I as claimed in claim 1, which comprises reacting a compound of the formula II 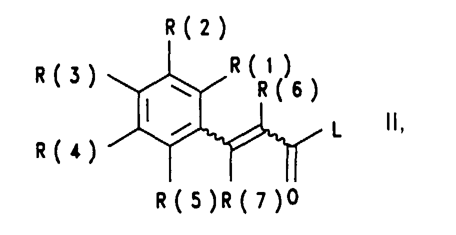 in which R(1) to R(7) are defined as in claim 1 and L is methoxy, phenoxy, phenylthio, methylthio, 2-pyridylthio, imidazolyl, -OCOOethyl, -O-SO₂-tolyl, -O-C(=N-cyclohexyl)-(NH-cyclohexyl) or -O-C+[N(CH₃)₂], with guanidine.

6. The use of a compound I as claimed in claim 1 for the preparation of a medicament for treatment of arrhythmias.

7. The use of a compound I as claimed in claim 1 for the preparation of a medicament for treatment or prophylaxis of cardiac infarction.

8. The use of a compound I as claimed in claim 1 for the preparation of a medicament for treatment or prophylaxis of angina pectoris.

9. The use of a compound I as claimed in claim 1 for the preparation of a medicament for treatment or prophylaxis of ischemic states of the heart.

10. The use of a compound I as claimed in claim 1 for the preparation of a medicament for treatment or prophylaxis of ischemic states of the peripheral and central nervous system and of apoplexy.

11. The use of a compound I as claimed in claim 1 for the preparation of a medicament for treatment or prophylaxis of ischemic states of peripheral organs and limbs.

12. The use of a compound I as claimed in claim 1 for the preparation of a medicament for treatment of states of shock.

13. The use of a compound I as claimed in claim I for the preparation of a medicament for use during surgical operations and organ transplants.

14. The use of a compound I as claimed in claim 1 for the preparation of a medicament for preservation and storage of transplants for surgical measures.

15. The use of a compound I as claimed in claim 1 for the preparation of a medicament for treatment of diseases of which cell proliferation is a primary or secondary cause.

16. A medicament which comprises an active content of a compound of the formula I as claimed in one or more of claims 1 to 3.

## Revendications

1. Guanidides d'acide cinnamique substitués de formule I 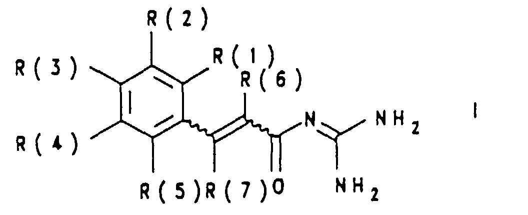 dans laquelle
au moins l'un des substituants R(1), R(2), R(3), R(4) et R(5) représente -Xₐ-Y_{b}-Lₙ-U,
X représente CR(16)R(17), O, S ou NR(18);
R(16), R(17) et R(18)
représentent, indépendamment les uns des autres, H ou un groupe alkyle ayant 1, 2, 3 ou 4 atomes de carbone, ou perfluoroalkyle ayant 1, 2, 3 ou 4 atomes de carbone;
a représente zéro ou 1;
Y représente un groupe alkylène ayant 1, 2, 3, 4, 5, 6, 7 ou 8 atomes de carbone, alkylène-T ayant 1, 2, 3, 4, 5, 6, 7 ou 8 atomes de carbone dans le fragment alkylène, T, T-alkylène ayant 1, 2, 3, 4, 5, 6, 7 ou 8 atomes de carbone dans le fragment alkylène;
T représentant un groupe NR(20), O, S ou phénylène,
le groupe phénylène étant non substitué ou portant de 1 à 3 substituants choisis dans l'ensemble constitué par F, Cl, CF₃, le groupe méthyle, le groupe méthoxy et
NR(21)R(22),
R(20), R(21) et R(22)
représentant, indépendamment les uns des autres, H ou un groupe alkyle ayant 1, 2, 3 ou 4 atomes de carbone ou perfluoroalkyle ayant 1, 2, 3 ou 4 atomes de carbone;
b représente zéro ou 1;
L représente O, S, NR(23) ou CₖH₂ₖ,
k représentant 1, 2, 3, 4, 5, 6, 7 ou 8;
n représente zéro ou 1;
U représente un groupe NR(24)R(25) ou un hétérocycle azoté ayant 1, 2, 3, 4, 5, 6, 7, 8 ou 9 atomes de carbone;
R(24) et R(25)
représentent, indépendamment l'un de l'autre, un atome d'hydrogène ou un groupe alkyle ayant 1, 2, 3, 4, 5, 6, 7 ou 8 atomes de carbone, perfluoroalkyle ayant 1, 2, 3, 4, 5, 6, 7 ou 8 atomes de carbone;
ou
R(24) et R(25)
représentent ensemble 4 ou 5 groupes méthylène, dont un groupe CH₂ peut être remplacé par un atome d'oxygène ou de soufre ou par un groupe NH, N-CH₃ ou N-benzyle;
les hétérocycles azotés étant liés par un pont N ou C et n'étant pas substitués ou portant de 1 à 3 substituants choisis dans l'ensemble constitué par F, Cl, CF₃, le groupe méthyle, le groupe méthoxy et NR(27)R(28),
R(23), R(27) et R(28)
représentant, indépendamment les uns des autres, H ou un groupe alkyle ayant 1, 2, 3 ou 4 atomes de carbone ou perfluoroalkyle ayant 1, 2, 3 ou 4 atomes de carbone;
et les autres substituants respectifs R(1), R(2), R(3), R(4) et R(5) représentent, indépendamment les uns des autres, H, F, Cl, Br, I, CN, -Oₙ-CₘH₂ₘ₊₁, -Oₚ-(CH₂)ₛ-C_{q}F_{2q+1} ou CᵣH₂ᵣR(10),
n représente zéro ou 1;
m représente zéro, 1, 2, 3, 4, 5, 6, 7 ou 8;
p représente zéro ou 1;
q représente 1, 2, 3, 4, 5, 6, 7 ou 8;
s représente zéro, 1, 2, 3 ou 4;
r représente zéro, 1, 2, 3 ou 4;
R(10) représente un groupe cycloalkyle ayant 3, 4, 5, 6, 7 ou 8 atomes de carbone, ou phényle,
le groupe phényle étant non substitué ou portant de 1 à 3 substituants choisis dans l'ensemble constitué par F, CI, CF₃, le groupe méthyle, le groupe méthoxy et NR(11)R(12),
R(11) et R(12)
représentant, indépendamment l'un de l'autre, H ou un groupe alkyle ayant 1, 2, 3 ou 4 atomes de carbone ou perfluoroalkyle ayant 1, 2, 3 ou 4 atomes de carbone;
R(6) et R(7) représentent, indépendamment l'un de l'autre,
un atome d'hydrogène, F, CI, Br, I, ou un groupe CN, alkyle ayant 1, 2, 3, 4, 5, 6, 7 ou 8 atomes de carbone, perfluoroalkyle ayant 1, 2, 3, 4, 5, 6, 7 ou 8 atomes de carbone, cycloalkyle ayant 3, 4, 5, 6, 7 ou 8 atomes de carbone, ou phényle
qui est non substitué ou porte de 1 à 3 substituants choisis dans l'ensemble constitué par F, Cl, CF₃, le groupe méthyle, le groupe méthoxy et NR(14)R(15),
R(14) et R(15)
représentant, indépendamment l'uns de l'autre, H ou un groupe alkyle ayant 1, 2, 3 ou 4 atomes de carbone ou perfluoroalkyle ayant 1, 2, 3 ou 4 atomes de carbone;
ainsi que leurs sels pharmaceutiquement acceptables.

2. Composé de formule I selon la revendication 1, caractérisé en ce que, dans ce composé:
au moins l'un des substituants R(1), R(2), R(3), R(4) et R(5) représente -Xₐ-Y_{b}-Lₙ-U,
X représente CR(16)R(17), O, S ou NR(18);
R(16), R(17) et R(18)
représentent, indépendamment les uns des autres, H, CH₃ ou CF₃;
a représente zéro ou 1;
Y représente un groupe alkylène ayant 1, 2, 3, 4, 5, 6, 7 ou 8 atomes de carbone, alkylène-T ayant 1, 2, 3, 4, 5, 6, 7 ou 8 atomes de carbone dans le fragment alkylène, T, T-alkylène ayant 1, 2, 3, 4, 5, 6, 7 ou 8 atomes de carbone dans le fragment alkylène;
T représentant un groupe NR(20), O, S ou phénylène,
le groupe phénylène étant non substitué ou portant de 1 à 3 substituants choisis dans l'ensemble constitué par F, CI, CF₃, le groupe méthyle, le groupe méthoxy et NR(21)R(22),
R(20), R(21) et R(22)
représentant, indépendamment les uns des autres, H, CH₃ ou CF₃
b représente zéro ou 1;
L représente O, S, NR(23) ou CₖH₂ₖ,
k représentant 1, 2, 3, 4, 5, 6, 7 ou 8;
n représente zéro ou 1;
U représente un groupe NR(24)R(25) ou un hétérocycle azoté ayant 1, 2, 3, 4, 5, 6, 7, 8 ou 9 atomes de carbone;
R(24) et R(25)
représentent, indépendamment l'un de l'autre, un atome d'hydrogène ou un groupe alkyle ayant 1, 2, 3, 4, 5, 6, 7 ou 8 atomes de carbone, perfluoroalkyle ayant 1, 2, 3, 4, 5, 6, 7 ou 8 atomes de carbone;
ou
R(24) et R(25)
représentent ensemble 4 ou 5 groupes méthylène, dont un groupe CH₂ peut être remplacé par un atome d'oxygène ou de soufre ou par un groupe NH, N-CH₃ ou N-benzyle;
les hétérocycles azotés étant liés par un pont N ou C et n'étant pas substitués ou portant de 1 à 3 substituants choisis dans l'ensemble constitué par F, CI, CF₃, le groupe méthyle, le groupe méthoxy et NR(27)R(28),
R(23), R(27) et R(28)
représentant, indépendamment les uns des autres, H, CH₃ ou CF₃;
et les autres substituants respectifs R(1), R(2), R(3), R(4) et R(5) représentent, indépendamment les uns des autres, H, F, CI, Br, I, CN, -Oₙ-CₘH₂ₘ₊₁, -Oₚ-(CH₂)_{d}-C_{q}F_{2q+1} ou -CᵣH₂ᵣR(10),
n représente zéro ou 1;
m représente zéro, 1, 2, 3 ou 4;
p représente zéro;
q représente 1, 2, 3, 4, 5, 6, 7 ou 8;
s représente zéro;
r représente zéro, 1 ou 2;
R(10) représente un groupe cycloalkyle ayant 3, 4, 5, 6, 7 ou 8 atomes de carbone, ou phényle, le groupe phényle étant non substitué ou portant de 1 à 3 substituants choisis dans l'ensemble constitué par F, Cl, CF₃, le groupe méthyle, le groupe méthoxy et
NR(11)R(12),
R(11) et R(12)
représentant, indépendamment l'un de l'autre, H, CH₃ ou CF₃;
R(6) et R(7) représentent, indépendamment l'un de l'autre, un atome d'hydrogène, F, Cl, Br, I, ou un groupe CN, alkyle ayant 1, 2, 3 ou 4 atomes de carbone, perfluoroalkyle ayant 1, 2, 3 ou 4 atomes de carbone, cycloalkyle ayant 3, 4, 5, 6, 7 ou 8 atomes de carbone, ou phényle
qui est non substitué ou porte de 1 à 3 substituants choisis dans l'ensemble constitué par F, Cl, CF₃, le groupe méthyle, le groupe méthoxy et NR(14)R(15),
R(14) et R(15)
représentant, indépendamment l'un de l'autre, H, CH₃ ou CF₃.

3. Composé de formule I selon la revendication 1 ou 2, caractérisé en ce que, dans ce composé:
au moins l'un des substituants R(2), R(3) et R(4) représente -Xₐ-Y_{b}-Lₙ-U,
X représente CR(16)R(17), O, S ou NR(18);
R(16), R(17) et R(18)
représentent, indépendamment les uns des autres, H, CH₃ ou CF₃;
a représente zéro ou 1;
Y représente un groupe alkylène ayant 1, 2, 3, 4, 5, 6, 7 ou 8 atomes de carbone, alkylène-T ayant 1, 2, 3, 4, 5, 6, 7 ou 8 atomes de carbone dans le fragment alkylène, T, T-alkylène ayant 1, 2, 3, 4, 5, 6, 7 ou 8 atomes de carbone dans le fragment alkylène;
T représentant un groupe NR(20), O, S ou phénylène,
le groupe phénylène étant non substitué ou portant de 1 à 3 substituants choisis dans l'ensemble constitué par F, Cl, CF₃, le groupe méthyle, le groupe méthoxy et
NR(21)R(22),
R(20), R(21) et R(22)
représentant, indépendamment les uns des autres, H, CH₃ ou CF₃
b représente zéro ou 1;
L représente O_{'} S, NR(23) ou CₖH₂ₖ,
k représentant 1, 2, 3, 4, 5, 6, 7 ou 8;
n représente zéro ou 1;
U représente un groupe NR(24)R(25) ou un hétérocycle azoté ayant 1, 2, 3, 4, 5, 6, 7, 8 ou 9 atomes de carbone;
R(24) et R(25)
représentent, indépendamment l'un de l'autre, un atome d'hydrogène ou un groupe alkyle ayant 1, 2, 3 ou 4 atomes de carbone ou CF₃;
ou
R(24) et R(25)
représentent ensemble 4 ou 5 groupes méthylène, dont un groupe CH₂ peut être remplacé par un atome d'oxygène ou de soufre ou par un groupe NH, N-CH₃ ou N-benzyle;
les hétérocycles azotés étant liés par un pont N ou C et n'étant pas substitués ou portant de 1 à 3 substituants choisis dans l'ensemble constitué par F, Cl, CF₃, le groupe méthyle, le groupe méthoxy et NR(27)R(28),
R(23), R(27) et R(28)
représentant, indépendamment les uns des autres, H, CH₃ ou CF₃;
et les autres substituants respectifs R(1), R(2), R(3), R(4) et R(5) représentent, indépendamment les uns des autres, H, F, Cl, Br, I, CN, -Oₙ-CₘH₂ₘ₊₁ ou CF₃,
n représente zéro ou 1;
m représente zéro, 1, 2, 3 ou 4;
R(6) et R(7) représentent, indépendamment l'un de l'autre, un atome d'hydrogène, F, Cl, Br, I, ou un groupe CN, alkyle ayant 1, 2, 3 ou 4 atomes de carbone, CF₃, cycloalkyle ayant 5, 6 ou 7 atomes de carbone, ou phényle
qui est non substitué ou porte de 1 à 3 substituants choisis dans l'ensemble constitué par F, Cl, CF₃, le groupe méthyle, le groupe méthoxy et NR(14)R(15),
R(14) et R(15)
représentant, indépendamment les uns des autres, H, CH₃ ou CF₃.

4. Composé de formule I selon l'une des revendications 1 à 3, caractérisé en ce qu'il est choisi dans l'ensemble constitué par les composés suivants:
guanidide d'acide E-3-(4-diméthylaminophényl)-2-méthylpropénoïque,
guanidide d'acide E-3-[4-(3-diméthylaminopropoxy)phényl)-2-méthyl-propénoïque,
guanidide d'acide E-3-[4-(3-pyridyloxy)-3-(trifluorométhyl)phényl]-2-méthyl-propénoïque,
guanidide d'acide E-3-[4-(4-pyridylthio)-3-(trifluorométhyl)phényl]-2-méthyl-propénoïque,
guanidide d'acide E-3-[3-cyano-4-diméthylamino-2-fluorophényl)-2-méthyl-propénoïque,
guanidide d'acide E-3-[4-(3-diméthylaminopropoxy)phényl]-2-fluoro-propénoïque,
guanidide d'acide E-3-[4-(4-2-diméthylaminoéthylène)phénoxy)phényl]-2-méthyl-propénoïque,
guanidide d'acide E-3-[3,5-difluoro-4-(4-(2-diméthylaminoéthylène)phénoxy)phényl]-2-méthyl-propénoïque,
guanidide d'acide E-3-[3,5-difluoro-4-(3-diméthylaminophénoxy)phényl]-2-fluoro-propénoïque,
guanidide d'acide E-3-[3,5-difluoro-4-(3-diméthylaminophénoxy)phényl]-2-méthyl-propénoïque,
guanidide d'acide E-3-[2,6-difluoro-4-(3-diméthylaminophénoxy)phényl]-2-méthyl-propénoïque,
guanidide d'acide E-3-[2,4-difluoro-6-(3-diméthylaminophénoxy)phényl]-2-méthyl-propénoïque.

5. Procédé pour la préparation d'un composé I selon la revendication 1, caractérisé en ce que l'on fait réagir avec de la guanidine un composé de formule II 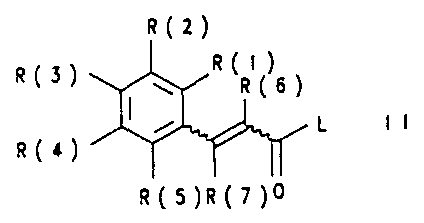 dans laquelle R(1) à R(7) sont tels que définis dans la revendication 1, et L représente le groupe méthoxy, phénoxy, phénylthio, méthylthio, 2-pyridylthio, imidazoyle, -OCOO-éthyle, -O-SO₂-tolyle, -O-C(=N-cyclohexyl)-(NH-cyclohexyle) ou -O-C⁺[N(CH₃)₂].

6. Utilisation d'un composé I selon la revendication 1, pour la fabrication d'un médicament destiné au traitement d'arythmies.

7. Utilisation d'un composé I selon la revendication 1, pour la fabrication d'un médicament destiné au traitement ou à la prophylaxie de l'infarctus du myocarde.

8. Utilisation d'un composé I selon la revendication 1, pour la fabrication d'un médicament destiné au traitement ou à la prophylaxie de l'angine de poitrine.

9. Utilisation d'un composé I selon la revendication 1, pour la fabrication d'un médicament destiné au traitement ou à la prophylaxie d'états ischémiques du coeur.

10. Utilisation d'un composé I selon la revendication 1, pour la fabrication d'un médicament destiné au traitement ou à la prophylaxie d'états ischémiques du système nerveux périphérique et central et de l'apoplexie cérébrale.

11. Utilisation d'un composé I selon la revendication 1, pour la fabrication d'un médicament destiné au traitement ou à la prophylaxie d'états ischémiques d'organes périphériques et des membres.

12. Utilisation d'un composé I selon la revendication 1, pour la fabrication d'un médicament destiné au traitement d'états de choc.

13. Utilisation d'un composé I selon la revendication 1, pour la fabrication d'un médicament destiné à l'utilisation dans des opérations chirurgicales et des greffes d'organes.

14. Utilisation d'un composé I selon la revendication 1, pour la fabrication d'un médicament destiné à la conservation et au stockage de transplants pour des opérations chirurgicales.

15. Utilisation d'un composé I selon la revendication 1, pour la fabrication d'un médicament destiné au traitement de maladies dans lesquelles la prolifération cellulaire représente une cause primaire ou secondaire.

16. Médicament, caractérisé par une teneur efficace en un composé de formule I selon une ou plusieurs des revendications 1 à 3.
